# EUROPEAN PATENT APPLICATION

(11) **EP 4 528 258 A1**
(43) Date of publication of application: **26.03.2025**
(21) Application number: 23807003.1
(22) Date of filing: 17.05.2023
(51) Int. Cl.: G01N 27/327, G01B 5/00

(54) **BIOSENSOR AND MANUFACTURING METHOD THEREFOR**

(30) Priority: 18.05.2022 CN 202210547593
(71) Applicant: Leadway (HK) Limited, Sheung Wan, Hong Kong (CN)
(72) Inventor: JI, Xubo, Hangzhou, Zhejiang 310030 (CN); LEI, Tianran, Hangzhou, Zhejiang 310030 (CN); WANG, Yi, Hangzhou, Zhejiang 310030 (CN); WU, Mengqun, Hangzhou, Zhejiang 310030 (CN); HUANG, Zhiheng, Hangzhou, Zhejiang 310030 (CN); GUO, Tao, Hangzhou, Zhejiang 310030 (CN); ZHANG, Li, Hangzhou, Zhejiang 310030 (CN)
(74) Representative: Schiweck Weinzierl Koch Patentanwälte Partnerschaft mbB
(86) International application number: PCT/CN2023/094858
(87) International publication number: WO 2023/222052

(57) **Abstract**

The present invention provides a biosensor and a preparation method thereof. The biosensor includes an insulating substrate and a conductive layer disposed on the insulating substrate, the conductive layer is divided by engraved lines to form electrodes, and an engraved line for identifying cutting deviation is disposed at least at a sample contacting end of the biosensor. According to the present invention, by providing the engraved line for identifying cutting deviation on the conductive layer, the cutting deviation during the cutting assembly process of the sensor can be found in time, ensuring the testing accuracy and stability of the prepared biosensor.

## Description

### Field of the Invention

The present invention relates to the field of biomedical testing, in particular to a biosensor and a preparation method thereof.

### Background of the Invention

Electrochemical sensors for detecting the content of an analyte in a sample and their matched detectors have been widely used in the daily monitoring of diseases. For example, diabetic patients commonly use electrochemical sensors to monitor daily glucose concentrations in the blood.

The basic structure of such electrochemical sensors includes an electrode system disposed on an insulating substrate, the electrode system includes a plurality of or multiple types of electrodes such as a working electrode and a counter electrode, and a reaction reagent that reacts with the analyte covers the corresponding electrodes. A sample interlayer having a channel is located on the electrode, a cover plate with an air hole covers the sample interlayer, the insulating substrate, the interlayer and the cover plate form a sample injection channel, and the other end of the electrode system is provided with a contact touching a contact pin of a detector. The sample flowing into the sample injection channel reacts with a reaction reagent on the electrodes to generate electrical signals from which the detector derives the test result.

The screen printing technology is utilized to print conductive materials on the insulating substrate to form electrodes, electrode contact pins and electrode leads, which is a method commonly used for preparing electrochemical sensors at present. When electrochemical sensors are manufactured using the screen printing method, large batch-to-batch variations exist. In order to eliminate the extent to which the batch-to-batch variations affect the test results, the problem of batch-to-batch variations is generally solved by inserting a calibration chip into the detector to correct the test results. However, inserting a calibration chip not only increases the operation steps of an operator, but also leads to inaccurate test results if the operator forgets to insert or incorrectly inserts the calibration chip. Some researchers also adopted, for example, the method in Chinese Patent ZL201210095096.5 to correct the batch-to-batch variations by setting calibration messages on the sensor.

In order to solve the problem of cumbersome testing steps or complicated preparation process mentioned above, Chinese Patent ZL00803756.6 provides a method for forming a thin film electrode, i.e., a thin film-like conductor layer is uniformly spread on an insulating substrate in advance, and then the electrode is formed by a laser etching method. This method has relatively high manufacturing precision and basically can ensure no batch-to-batch variations between products produced in different batches, or that the presence of batch-to-batch variations will not affect the test results in the case where no correction is required.

For such sensor with electrodes formed by the laser ablation method, when assembling a large layout (a semi-finished product) composed of a plurality of basic unit sensors into an independent sensor (finished products) in its production process, it needs to ensure in the production process that the provided electrodes will not result in deviated electrode areas due to the unstable assembly process, and the area specifically refers to the area on which the substance to be tested can generate an electrical signal or other electrode area on which an electrical signal is conducted during the sensor test process. Therefore, the present invention is completed based on the above problems, and has an objective of providing a simple design which can find the deviation of cutting assembly in time, so as to solve the above problems.

### Summary of the Invention

The present invention is completed based on the above problems in the prior art, and has an objective of providing a biosensor which can find the deviation of cutting assembly in time.

The biosensor described in the present invention is a novel biosensor, which comprises an insulating substrate and a uniform conductive layer disposed on the insulating substrate, electrodes being formed on the conductive layer and divided by engraved lines. A reagent that can specifically test a substance to be tested is allocated to all or part of the electrodes at a sample contacting end, and the electrodes and the reagent are provided with a fluid channel for entrance of the solution of a substance to be tested. The electrodes extend from the sample contacting end to a contact end of the sensor and conduct the electrical signal generated by the reaction of the substance to be tested with the reagent or other electrical signal required in the test process of the sensor to the contact end of the sensor. In this process, large deviation of the electrode areas of different sensors will lead to fluctuations of the electrical signal and thus generate deviation of the test results. In order to prevent the deviation of the electrode area caused by the deviation of cutting assembly, an electrode is provided with an engraved line for identifying cutting deviation that can efficiently determine whether the cutting deviation is present or not. When the engraved line for identifying cutting deviation can be clearly observed on the product after the cutting assembly or when the engraved line for identifying cutting deviation is still complete, the product can be determined as a qualified product. Because of the design of the engraved line for identifying cutting deviation, it is possible to easily and accurately screen out the biosensor of which the electrode area has deviation due to cutting assembly errors.

The biosensor described in the first aspect of the present invention is characterized in that, in the biosensor described in the first aspect, the sample to be tested enters from the sample contacting end of the biosensor, and fills in the fluid channel to react with a reagent on all or part of the electrodes to generate an electrical signal. The electrodes comprise a first test electrode, a second test electrode, and/or a third test electrode, a test instrument generates an electrical feedback signal by applying an electrical signal or generates an electrical feedback signal by applying an electrical signal to promote the reaction of the substance to be tested with the reagent on the electrodes, and the electrical feedback signal is conducted back to the test instrument via the electrodes to achieve quantitative testing. The sizes of the corresponding first test electrodes, second test electrodes, and third test electrodes of the biosensors constituted in such a way should have no deviation in order to obtain a good response, so that in the present invention, it precisely specifies that the electrode size at the sample contacting end will not fluctuate due to cutting assembly errors, by providing the engraved line for identifying cutting deviation between the end edge at the sample contacting end and the electrode.

The biosensor described in the second aspect of the present invention is characterized in that, in the biosensor described in the second aspect, the engraved line for identifying cutting deviation disposed between the sample contacting end and an electrode and the engraved line for forming the electrode by division are the same engraved line, at which point the engraved line for identifying cutting deviation is located between the end edge at the sample contacting end and the electrode and is adjacent to the electrode. Due to the design of such an electrode structure, the number of the engraved lines can be reduced and the distance between the end edge at the sample contacting end and the electrode can be shortened, which can save laser processing time and has the effect of biosensors with uniform performance.

The biosensor described in the third aspect of the present invention is characterized in that, in the biosensor described in the first and second aspects, the provided engraved line for identifying cutting deviation is an irregular or discontinuous curve, e.g., an arc. Due to the design of such electrode structure, it is easier for a production people to identify the level of completeness of the engraved line for identifying cutting deviation when carrying out interpretation and authentication, so that the effect of biosensors with uniform performance can be achieved.

Specifically, the present invention provides a biosensor, comprising an insulating substrate and a uniform conductive layer disposed on the insulating substrate, the conductive layer is divided by engraved lines to form electrodes, and an engraved line for identifying cutting deviation is disposed at least at the sample contacting end of the biosensor.

Further, the engraved line for identifying cutting deviation is located between the end edge of the conductive layer at the sample contacting end and the electrode adjacent to the end edge of the conductive layer at the sample contacting end.

Further, the electrode refers to the electrode participating in the conduction of an electrical signal in the biosensor.

Further, an engraved line for identifying cutting deviation is disposed between the end edge of the conductive layer at the sample contacting end and the electrode closest to the end edge of the conductive layer and participating in the conduction of electrical signals.

Further, the engraved line for identifying cutting deviation intersects or does not intersect side edges of the conductive layer.

Further, the engraved line for identifying cutting deviation is a straight line parallel to the end edge of the conductive layer.

Further, the engraved line for identifying cutting deviation is a curve.

Further, the engraved line for identifying cutting deviation does not participate in formation of the electrode.

Further, the engraved line for identifying cutting deviation participates in formation of the electrode, and the electrode is adjacent to the end edge of the conductive layer at the sample contacting end.

Further, the conductive layer is a gold film, a palladium film, an alloy film or a carbon film.

Further, the width of the engraved lines is within the range of 0.020mm to 0.200mm. Further, the electrode system comprises a working electrode, a counter electrode and a reference electrode.

The present invention also provides a preparation method of a biosensor, comprising:
step 1: according to a pre-designed electrode pattern, performing laser etching on an original material having an insulating substrate and a conductive layer to etch the electrode pattern;
step 2: adding a test reagent to corresponding electrodes requiring addition;
step 3: affixing an interlayer and an upper cover on the electrodes, and forming a sample injection channel at the sample injection end of the biosensor to obtain a semi-finished large card; and
step 4: cutting the semi-finished large card by using a cutter along a preset cutting line to obtain finished independent biosensor,
wherein the engraved line for identifying cutting deviation is etched at the sample injection end of the biosensor in step 1.

Further, an electrode pattern is etched using laser etching.

Further, engraved lines are formed after etching.

Further, the conductive layer is selected from a gold film, a palladium film, an alloy film or a carbon film.

Further, the width of the engraved lines is within the range of 0.020mm to 0.200mm.

Beneficial effects: according to the present invention, by providing the engraved line for identifying cutting deviation on the conductive layer, the cutting deviation during the cutting assembly process of the sensor can be found in time, ensuring the testing accuracy and stability of the prepared biosensor.

### Brief Description of the Drawings

Fig. 1 is an exploded oblique view of a biosensor in the solution of a first example.
Fig. 2 is a front view of an electrode system on a conductive layer corresponding to the biosensor in the solution of the first example.
Fig. 3 is a flow of a method for manufacturing the biosensor in the present invention.
Fig. 4a is a schematic diagram of the head-to-head and tail-to-tail sequential arrangement of sensor base units A obtained by etching the conductive layer.
Fig. 4b is a schematic diagram of the co-directional arrangement of three rows of sensor base units A obtained by photo-etching the conductive layer.
Fig. 5a is a schematic diagram of the tail-to-tail arrangement of the sensor base units A obtained by etching the conductive layer.
Fig. 5b is a schematic diagram of the head-to-head arrangement of the sensor base units A obtained by etching the conductive layer.
Fig. 6 is a schematic diagram of a semi-finished strip-shaped sheet formed by cutting a semi-finished large card.
Fig. 7 is a front schematic diagram of an electrode system provided with no engraved line for identifying cutting deviation.
Fig. 8 is a front schematic diagram of an electrode system provided with an engraved line for identifying cutting deviation in the solution of the first example.
Fig. 9 is a front schematic diagram of an electrode system provided with a second form of engraved line for identifying cutting deviation in the solution of the first example.
Fig. 10 is a front schematic diagram of the arrangement of one row of the sensors in Fig. 9.
Fig. 11 is an exploded oblique view of a sensor described in the solution described in a second example.
Fig. 12 is a front schematic diagram of an electrode system of the sensor in the solution described in the second example.
Fig. 13 is a schematic diagram of the arrangement of one row of electrode systems of a sensor provided with a second form of engraved line for identifying cutting deviation in the second example.
Fig. 14 is a front schematic diagram of an electrode system of a sensor provided with an arc-shaped engraved line for identifying cutting deviation in a third example.
Fig. 15 is a front schematic diagram of an electrode system of a sensor provided with an arc-shaped extended engraved line for identifying cutting deviation in the third example.
Figs. 16a and 16b are front views of an electrode system of a sensor described in the solution of a fourth example.
Fig. 17 is a line graph of glucose sensor test values versus YSI test values of a fifth example.

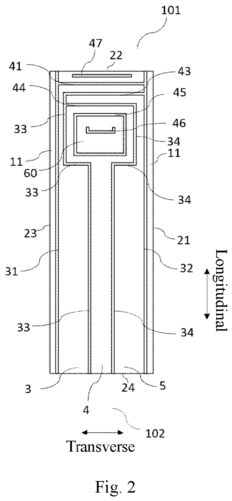

In order to clearly show the electrode and the engraved lines, the conductive layer is not shown as a profile line in Figs. 2, 4a, 4b, 5a, 5b, Figs. 6 to 9, and Fig. 12 to Figs. 16a and 16b.

### Detailed Description of the Embodiments

Embodiments of the present invention will be described below with reference to the drawings. The listed embodiments are merely a few of the examples of the present invention, and the technical solution of the present invention is not limited thereto.

### Example 1:

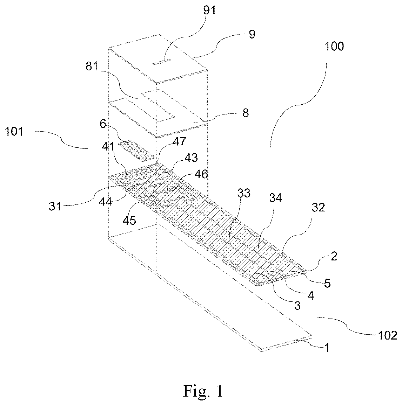

Fig. 1 and Fig. 2 are a biosensor 100 in the first aspect of Example 1 of the present invention.

The biosensor 100 as shown in Fig. 1 includes an insulating substrate 1, and an electrode system disposed on the insulating substrate 1 and formed through dividing a conductive layer 2 via engraved lines. The electrode system includes a working electrode 5, a counter electrode 4 and a reference electrode 3, and the electrodes have a sample contacting end 101 contacting a sample and a contact end 102 contacting a contact pin of an analyzer. A reagent layer 6 is added to corresponding electrodes at the sample contacting end. An interlayer 8 with an open groove 81 covers the sample contacting end of the electrodes, and an upper cover 9 covers the interlayer 8, so that the conductive layer 2, the open groove 81 and the upper cover 9 form a fluid channel for entrance of a fluid sample under test. The electrodes and the reagent layer are in the channel. The upper cover 9 is provided with an air hole 91, which is located above the open groove and used for discharging air in a sample injection channel after sample addition.

Fig. 2 is a front view of the conductive layer 2 in Fig. 1. The engraved lines are insulating gaps left after removal of a conductive material from the conductive layer 2. As shown in Fig. 2, the working electrode 5 is enclosed by engraved lines 32, 33, 34, 43, 44 and an edge line 24 of the conductive layer , the counter electrode 4 is enclosed by engraved lines 33, 34, 44, 45 and the end edge 24 of the conductive layer, and the reference electrode 3 is enclosed by engraved lines 31, 32, 33, 41, 43 and the edge line 24 of the conductive layer.

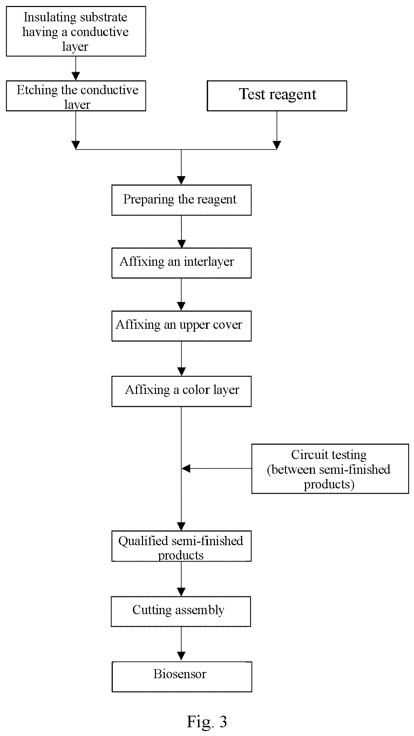

A method for manufacturing the biosensor shown in Fig. 1 is illustrated by taking Figs. 3, 4, 5 and 6 as examples.

Step 1: laser etching of an electrode pattern: according to a pre-designed electrode pattern, on an original material having an insulating substrate and a conductive layer, etching the electrode pattern with laser on the conductive layer, thus obtaining a large electrode card with a plurality of sensor base units A.

Step 2: addition of the reagent layer: preparing a test reagent, and adding the prepared reagent to corresponding electrodes requiring addition of the reagent.

Step 3: affixing the interlayer 8 to each sensor base unit A. Generally, the interlayer is placed at the sample contacting end of the biosensor.

Step 4: sticking the upper cover 9 to each interlayer 8 and rolling.

Step 5: after the upper cover is affixed, affixing and rolling a color layer on the upper cover to obtain a semi-finished large card.

Step 6: cutting: cutting the semi-finished large card by using a cutter along a preset cutting line to obtain finished biosensors.

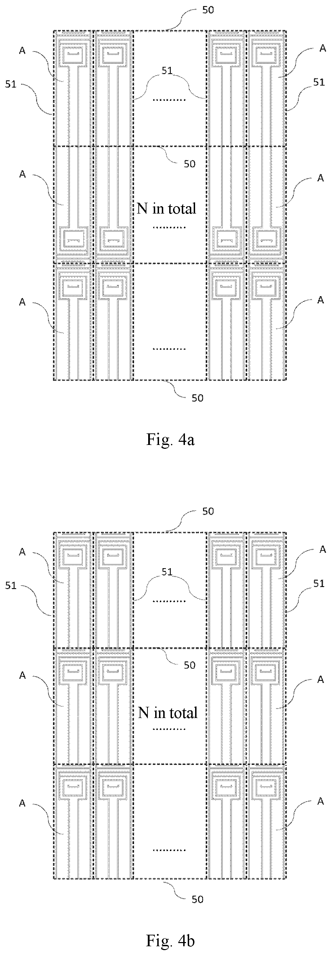

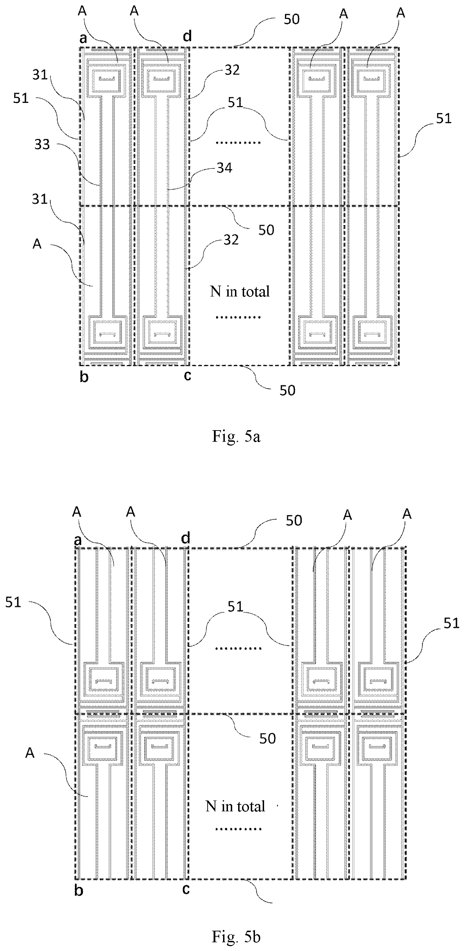

The manufacturing steps are specifically illustrated by taking Fig. 4a, Fig. 4b, Fig. 5a or Fig. 5b as an example. In step 1, the conductive layer 2 on the surface of the insulating substrate is etched using the laser etching technology to form a plurality of engraved lines 31 to 34 and engraved lines 41 to 47 on the conductive layer 2, and the conductive layer in these engraved lines is basically cleared away to expose the insulating substrate. Through division by the engraved lines, the working electrode 5, the counter electrode 4, the reference electrode 3, and other engraved lines with certain uses are formed on the conductive layer. After laser etching is completed, a large electrode card is obtained, including a plurality of sensor base units A on an insulating substrate, which are arranged side by side and can be used to manufacture finished biosensors. Each sensor base unit A includes an insulating substrate, an electrode system formed after division by engraved lines, the engraved lines, and other blocks formed by etching. In this example, the electrode system includes the working electrode 5, the counter electrode 4, and the reference electrode 3. Fig. 4a shows three rows of sensor base units A arranged in sequence head to head and tail to tail, which are formed by etching; and Fig. 4b shows three rows of sensor base units A in co-directional arrangement, which are formed by laser etching. Fig. 5a shows two rows of sensor base units A arranged in sequence tail to tail, which are formed by etching; and Fig. 5b shows two rows of sensor base units A arranged head to head, which are formed by etching, wherein N represents 0 to N; and "N in total" represents inclusion of N sensor base units A. The number of rows and the number of columns here are merely illustrative, and both the number of rows and the number of columns can be more than two.

In step 1, i.e., laser etching of an electrode pattern, the etching travel line of the engraved lines within the conductive layer is controlled by software. Taking Fig. 5a as an example, the engraved line 31 is formed in such a way that continuous laser cutting is completed by starting from point a on the conductive layer to point b via the plurality of sensor base units A on the conductive layer. Similarly, the engraved line 32 is formed in such a way that continuous laser cutting is completed by starting from point c to point d via the plurality of sensor base units A on the conductive layer. The specific implementation of the path and direction and the like of the engraved lines can be artificially designed and controlled by software according to the needs of actual products, and is not limited to the above path. Suitable laser etching parameters are used for the engraved lines, e.g., the width of the engraved lines is within the range of 0.020mm to 0.200mm, and the width of the engraved lines used in this example is 0.080mm.

Fig. 4a, Fig. 4b, Fig. 5a and Fig. 5b are just four of the forms formed after step 1, and there may be other forms of arrangements as well, as long as the sensor base units A arranged regularly or irregularly can be cut effectively in the back-end process. It is also possible to use Fig. 4a or Fig. 5a as a unit, and the large electrode card formed after etching is an arrangement of a plurality of units shown in Fig. 4a or Fig. 5a, for example, if Fig. 4a is used as a repeating unit, N is 4 and the times of repetition is 2, the manufactured semi-finished product may have six rows and four columns, and for another example, if Fig. 5a is used as a repeating unit, N is 10 and the times of repetition is 4, the manufactured semi-finished product may have eight rows and ten columns in total.

The material of the insulating substrate is polyvinyl chloride, polyterephthalic acid, polyethylene terephthalate, macrogol ester, etc., with polyethylene terephthalate being preferred in this example.

The material of the conductive layer 2 may be gold, silver, platinum, palladium, carbon, graphite, conductive glass, and other conductive metal or conductive non-metal not limited thereto, or a mixture thereof. The method for covering the conductive layer may use printing, coating, electroplating, sputtering, etc., and this example uses a conductive carbon layer (carbon film) formed in a coating manner, the thickness of the carbon layer is 1-30um, the thickness used in this example is about 8um, and the resistance of the conductive layer is 10 Ω/□ to 100 Ω/□, preferably 30 Ω/□ in this example.

Some or all of the electrodes of the biosensor may be configured with a reagent layer. The reagent layer employs a specific enzyme to quantitatively or qualitatively analyze the measured target substance under the environment of a buffer system, generally containing the following components: enzyme, electronic mediator, high polymer, disintegrant, surfactant, stabilizer and buffer system. Depending on the needs of testing items, the enzyme is selected from one or more of glucose oxidase, glucose dehydrogenase, lactate oxidase, lactate dehydrogenase, urate oxidase, cholesterol oxidase or D-3-hydroxybutyrate oxidase, etc.; the electronic mediator is selected from one or more of ruthenium compounds, potassium ferricyanide, ferrocene, etc.; the buffer system is any one or more of sodium succinate, sodium citrate, piperazine buffer, propanesulfonic acid, PBS buffer or sodium fumarate; the disintegrant is any one or more of crosslinked PVP, sodium carboxymethyl starch, or crosslinked CCNa; the surfactant is any one or more of anionic surfactant, cationic surfactant, diatomic surfactant, or nonionic surfactant; and the stabilizer is one or more of maltitol, trehalose, BSA, or protein protective agent. The above components after preparation are stirred sufficiently, so that the components are fully dissolved and dispersed to form a homogeneous solution. In this example, the biosensor as shown in Fig. 1 includes a reagent layer 6.

In step 2 for preparing the reaction reagent, methods such as dispensing, screen printing, drop coating and Slot-Die coating are usually adopted to configure a reagent mixture having certain chemical components onto a specific electrode of the biosensor to form the reagent layer. It is preferred in this example that screen printing is used to configure a biochemical reagent on the specific electrode in specific pattern and position. The biochemical reagent in the specific pattern and position can react with the substance to be tested in the sample and generate a certain electrical signal, and the electrical signal is conducted to a test instrument and fed back to a user via a conductive material. The screen printing plate used in the screen printing generally employs polyester, nylon, stainless steel and other materials, and is generally of 250 meshes to 420 meshes, the used wire screen has a wire diameter of 27um to 120um generally, and bears a maximum tension of 22 to 38N/cm generally. It is preferred in this example that a 305-mesh nylon screen printing plate with a wire diameter of 34um is used, which can bear a maximum tension of 33N/cm.

The above biochemical reagent with fixed pattern and position obtained via screen printing comes into contact with the sample in the sample injection channel and reacts with the substance to be tested in the sample. The sample injection channel is formed between the open groove 81 and the upper cover 9, and the surface of the upper cover facing the sample injection channel is a hydrophilic layer material. Specifically, the material of the interlayer is typically a PET substrate coated with an acrylic resin system as an adhesive material. The thickness of the interlayer is typically 75um to 150um, and the width of the open groove is typically 0.7mm to 1.8mm. In this example, the thickness of the interlayer is preferably 100um, and the width of the hollow structure is preferably 1.2mm.

An air hole 91 is left in the upper cover 9 at the tail of the sample injection channel, and the air hole may be round, square, rectangular, linear, or in other shapes. It is preferred in this example that the air hole is rectangular. In this example, the hydrophilic material is preferably 9901P made by 3M. The air hole of the hydrophilic material can ensure that the original air in a chamber is smoothly discharged when a blood sample flows into the sample injection channel, so as to ensure that the sample can smoothly flow into the chamber.

The color layer is generally a printable single-sided adhesive, with the product name generally printed on the surface, which plays a role of easily identifying the product and protecting the reagent strip from damage caused by scratching.

Step 5 is a step that can be omitted. For example, if the upper cover itself can be printed with product name, which can simultaneously serve a similar function as the color layer, step 5 is omitted. Alternatively, if the biosensor does not require the color layer, step 5 is omitted.

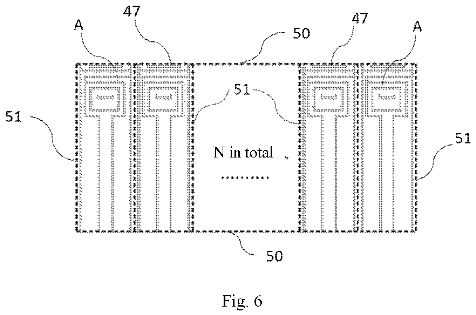

In the cutting step 6, the semi-finished large card is cut using a cutter along a preset cutting line, specifically, using the cutter along the cutting lines at positions shown by dotted lines 50 and dotted lines 51 in Fig. 4a and Fig. 5a, to obtain finished biosensors. Suitable cutting methods include but not limited to hobbing, die punching, chopping, etc. Specifically, it is preferred in this example to use a hob to cut the semi-finished large card as shown in Figs. 4a and 5a along the horizontal dotted line 50 into semi-finished strip-shaped sheets containing several sensor base units A as shown in Fig. 6, and then use the hob to cut the semi-finished strip-shaped sheets along the vertical dotted lines 51 into finished biosensors. In order to more visually illustrate the cutting position of the hob, when Figs. 4a, 5a, and 6 are used to introduce the cutting process of step 6, the interlayer and the upper cover are not shown in Figs. 4a, 5a and 6. The cutting process described in step 6 is one of the most important steps in the assembly of the sensor, the effect of which directly affects the pass rate of the final finished sensors and the finished product effect of the sensors. The dotted lines labeled in Fig. 4a and Fig. 5a are not present in the actual product processing, the same below.

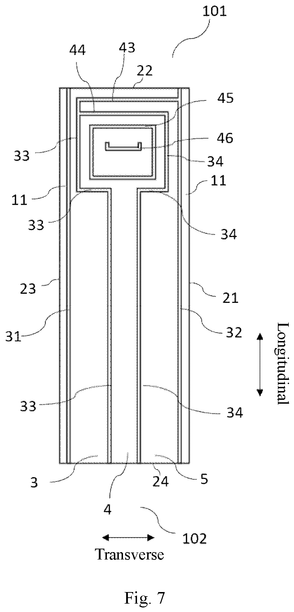

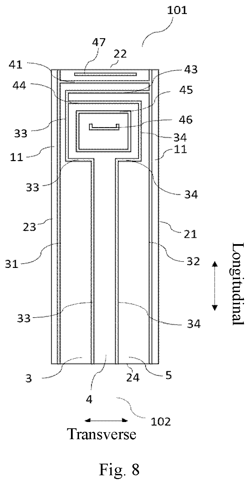

The biosensors in Figs. 7 and 8 are taken examples below to specifically introduce how Fig. 8 of the example of the present invention ensures the pass rate of the finished biosensor products in the cutting assembly process.

A biosensor includes an electrode system on a conductive layer and an engraved line layout as shown in Fig. 7. The electrode system and engraved line layout of the biosensor are different from those in Fig. 1, and the other structures are the same as those in Fig. 1 of Example 1. As shown in Fig. 7, the working electrode 5 is enclosed by engraved lines 32, 33, 34, 43, 44 and an edge line 24 of the conductive layer, the counter electrode 4 is enclosed by engraved lines 33, 34, 44, 45 and the edge line 24 of the conductive layer, and the reference electrode 3 is enclosed by engraved lines 31, 32, 33, 43 and the edge lines 22, 24 of the conductive layer. Specifically, the edge lines 22, 24 of the conductive layer are obtained by cutting the semi-finished large card along the cutting lines 50. The biosensor is provided with a reagent on the electrodes 3, 4, 5 at the sample contacting end, and the electrodes on the conductive layer, the open groove 81 of the interlayer and the upper cover 9 together constitute a fluid channel for entrance of the sample to be tested, wherein the air hole 91 formed in the upper cover ensures air discharge after the sample to be tested enters the channel. After the sample to be tested enters the channel, a test instrument applies a certain electrical signal to the electrodes 3, 4, 5 so that the tested sample reacts with the reagent disposed on the electrodes and generates an electrical feedback signal, which is then conducted back to the test instrument via the electrodes 3, 4, 5 to achieve the testing of the sample to be tested.

More specifically, the sizes of the electrodes 3, 4 and 5 that contact the sample to be tested at the sample contacting end must be very accurate, and the sizes of similar electrodes of biosensors of the same model and different batch numbers should be the same or basically the same within the allowable range of the design. If the electrode sizes deviate largely, it will definitely lead to different electrical feedback signals generated and conducted by the sensors of different batch numbers, and will inevitably result in fluctuations of the test values of the test instrument.

The electrodes 4 and 5 are divided by the engraved lines on the conductive layer, and the electrode sizes thereof are controlled by a laser cutting instrument and software jointly and extremely high in accuracy, so that it can be considered that the electrode sizes between different batches of electrodes 4 and electrodes 5 have no fluctuation. The electrode 3 is formed by the engraved lines together with the edge lines 22, 24 of the conductive layers, and is the electrode with the closest physical distance from the edge line 22 of the conductive layer at the sample contacting end. Deviation generated from factors such as manual work in the cutting assembly process will directly affect the positions and effects of the edge lines 22, 24 of the conductive layer.

Specifically, if fluctuations resulted from human operation in the cutting assembly process cause inaccurate cutting of a cutter at the preset cutting line 50, it will result in simultaneous upward deviation, downward deviation, or skewing of the edge lines 22 and 24 of the conductive layer. As shown in Fig. 7, the edge line 24 of the conductive layer is at the sensor contact end of the electrode 3, and fluctuations in the position thereof do not affect the signal conduction of the electrical feedback signal to the test instrument; while the edge line 22 of the conductive layer is at the sample contacting end of the electrode 3, and fluctuations thereof directly lead to uncontrollable fluctuations in the area of the electrode 3 that is in contact with the sample to be tested, which will lead to fluctuations in the test values between different batches of sensors. With respect to the situation in which the structure of Fig. 7 above may cause the size of the cut electrode 3 to be inconsistent with the preset value, the present invention proposes the solution of Fig. 8, which can avoid the above risk. Specifically as shown in Fig. 8, the specific composition of the electrodes 4, 5 obtained by division on the conductive layer is not changed, while the electrode 3 is enclosed by the engraved lines 31, 32, 33, 41, 43 and the edge line 24 of the conductive layer, and an engraved line 47 is disposed between the edge line 22 of the conductive layer located at the sample contacting end and the electrode 3. The position of the engraved line 47 on the semi-finished strip-shaped sheet is as shown in Fig. 6.

Specifically in conjunction with Fig. 6, during the traveling process of the manually operated cutter along the cutting line 50, fluctuations due to the manual operation cause simultaneous upward deviation, downward deviation or skewing of the edge lines 22, 24 of the conductive layer of the sensor. The finished sensor obtained after cutting can be interpreted by observing the shape or presence/absence of the engraved line 47 on the obtained finished sensor to determine whether the sensor is a qualified product. More specifically, the engraved line 47 is an engraved line for identifying cutting deviation, and when a complete engraved line 47 for identifying cutting deviation can be seen in the obtained finished sensor, the electrode 3 closest to the edge line 22 of the conductive layer at the sample contacting end is certainly complete and not affected by manual cutting errors. By the quality inspection process of interpreting the engraved line 47 for identifying cutting deviation, the product quality during the cutting assembly process can be effectively guaranteed, and the sensor with the carving line 47 not meeting requirements is determined as an unqualified product, so that the sizes of various electrodes in the sensor can be simply and accurately guaranteed, thereby ensuring the accuracy of the product performance.

The situation of the engraved line 47 can be observed through the fluid channel of the finished sensor. Specifically, the upper cover of the biosensor is made of a transparent material, and if a color layer is attached to the upper cover, the position on the color layer corresponding to the fluid channel is of an evacuated design or has no color, and then whether the finished sensor is a qualified product without cutting deviation can be determined by observing the situation of the engraved line 47 in the fluid channel.

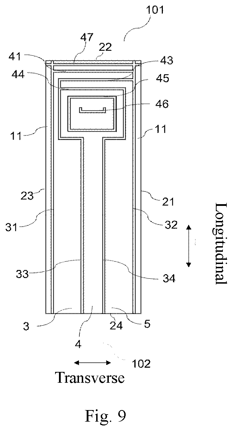

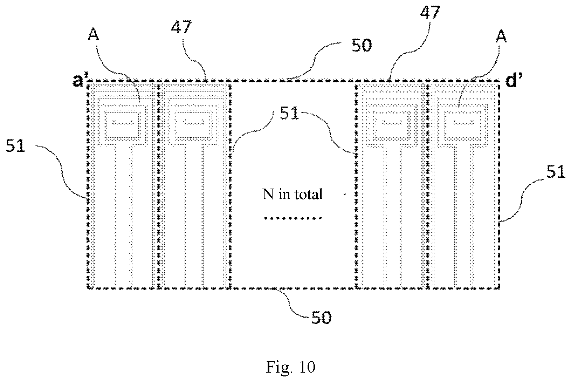

Fig. 9 is an improved design on the basis of Fig. 8. Further introduction will be given below by takings Figs. 9 and 10 as an example.

As shown in Fig. 9, the engraved line 47 for identifying cutting deviation located between the edge line 22 of the conductive layer at the sample contacting end and the electrode 3 extends to both sides in the transverse direction of the sensor. In the semi-finished strip-shaped sheet as shown in Fig. 10, the etching path of the engraved line 47 for identifying cutting deviation of a plurality of base units A may travel consecutively. Specifically, in the plurality of base units A, the etching path of the engraved line 47 for identifying cutting deviation starts from point a' of the 1st base unit A and finally ends at point d' of the Nth base unit A via the engraved line 47 for identifying cutting deviation of the N base units A. The continuous etching on the insulating substrate and conductive layer of the original material reduces the number of times of jumping of the laser travel path in the laser etching process, and more specifically, can achieve the optimization of the laser etching production process for the sensor, improve the production efficiency and reduce the time loss.

The original material described in the present invention refers to a material that has an insulating substrate and a conductive layer and is not etched by laser. The large electrode card refers to a material that the laser-etched original material has a full-layout electrode pattern. The semi-finished large card refers to a material that a reagent layer, an interlayer and an upper cover are added to the large electrode card. The semi-finished strip-shaped sheet refers to a strip-shaped material obtained from cutting the semi-finished large card is cut by a large hob. The finished biosensor refers to a sensor 100 which is obtained from cutting the semi-finished strip-shaped sheet by a small hob and can be used alone. The plurality of sensor base units A are formed on the large electrode card via the electrode pattern and the engraved lines, and the reagent layer, the interlayer, the upper cover and the like are added to the sensor base units A, and cutting is performed to form sensors used alone.

### Example 2

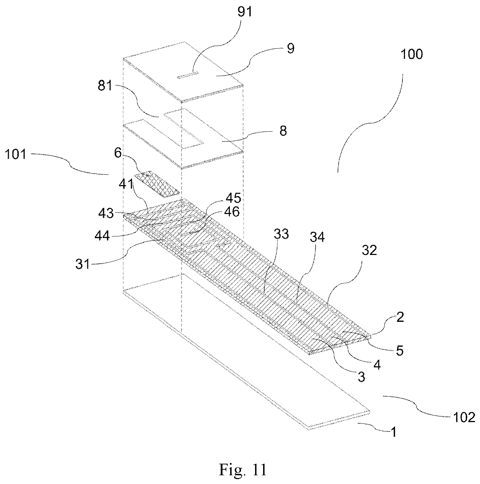

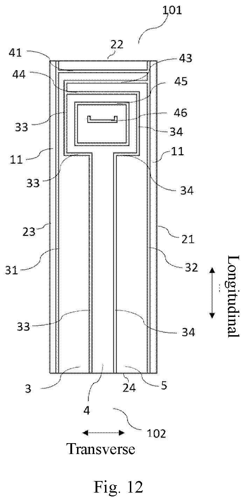

The biosensor 100 in Example 2 of the present invention is illustrated by taking Figs. 11 and 12 as an example.

Fig. 11 shows an exploded view of the sensor described in Example 2, and Fig. 12 shows a front view of an electrode system of the conductive layer. The working electrode 5 is enclosed by engraved lines 32, 33, 34, 43, 44 and the edge line 24 of the conductive layer, the counter electrode 4 is enclosed by engraved lines 33, 34, 44, 45 and the edge line 24 of the conductive layer, and the reference electrode 3 is enclosed by engraved lines 31, 32, 33, 41, 43 and the edge line 24 of the conductive layer. Specifically, the edge line 24 of the conductive layer is obtained by cutting the semi-finished large card along the cutting lines 50. A reagent is disposed on the electrodes 3, 4, 5 at the sample contacting end, and the electrodes on the conductive layer, the open groove 81 of the interlayer and the upper cover 9 jointly constitute a fluid channel for entrance of the sample to be tested, wherein the air hole 91 formed in the upper cover ensures air discharge after the sample to be tested enters the channel. After the sample to be tested enters the channel, a test instrument applies a certain electrical signal to the electrodes 3, 4, 5 so that the tested sample reacts with the reagent disposed on the electrodes and generates an electrical feedback signal, which is then conducted back to the test instrument via the electrodes 3, 4, 5 to achieve the testing of the sample to be tested.

More specifically, the sizes of the electrodes 3, 4, 5 that contact the sample to be tested at the sample contacting end must be very accurate, and the electrode sizes between sensors of different batch numbers should not fluctuate, and if fluctuations exist, it will definitely lead to different electrical feedback signals generated and conducted by the sensors of different batch numbers, and will inevitably result in fluctuations of the test values of the test instrument.

The electrodes 4 and 5 are divided by the engraved lines on the conductive layer, and the electrode sizes thereof are controlled by a laser cutting instrument and software jointly and extremely high in accuracy, so that it can be considered that the electrode sizes between different batches of electrodes 4 and electrodes 5 have no fluctuation. The electrode 3 described in the aforementioned Example 1 may fluctuate in electrode size due to manual cutting deviation. In the aforementioned Example 1, the engraved line 47 for identifying cutting deviation is disposed on the electrode system in the present invention, and more specifically, the engraved line for identifying cutting deviation is disposed between the edge line 22 of the conductive layer at the sample contacting end and the electrode 3, and the finished sensor obtained after cutting can be interpreted by observing the shape or presence/absence of the engraved line 47 on the obtained finished sensor to determine whether the sensor is a qualified product. When a complete engraved line 47 for identifying cutting deviation can be seen in the obtained finished sensor, it can be determined that the electrode 3 closest to the edge line 22 of the conductive layer at the sample contacting end is certainly complete and not affected by manual cutting errors. By the quality inspection process of interpreting the engraved line 47 for identifying cutting deviation, the product quality during the cutting assembly process can be effectively guaranteed, and the sizes of various electrodes in the sensor can be simply and accurately guaranteed, thereby ensuring the accuracy of the product performance.

Example 2 is on the basis of Example 1, the engraved line 47 for identifying cutting deviation in this example shares the same engraved line with the engraved line 41 enclosing the electrode 3, that is, the engraved line 41 is not only used as the engraved line for formation of the electrode 3, but also used as the engraved line for identifying cutting deviation. More specifically, at the sample contacting end, the engraved line for identifying cutting deviation is disposed between the edge line of the conductive layer and the electrode, and is adjacent to the electrode. In the cutting assembly process of the semi-finished large card, a quality inspector only needs to determine the engraved line 41 adjacent to the electrode 3 on the obtained finished sensor, and when the engraved line 41 is complete and not affected by cutting deviation, it can be considered that the electrode 3 is not affected by cutting errors, i.e., the size of the electrode 3 is not affected. The solution is simpler than Example 1.

Compared to Example 1, the solution of Example 2 may further reduce the number of engraved lines in the electrode system, and further reduce the difficulty and required processing time of laser etching in the process of obtaining the large electrode card by laser etching. More preferably, the engraved line for identifying cutting deviation is disposed to be adjacent to the electrode, which can reduce the distance between the electrode and the edge line of the conductive layer. In other words, the electrode system can get close to the edge line 22 of the conductive layer at the sample contacting end as a whole, and accordingly, the interlayer 8 and the open groove 81 therein, and the upper cover 9 and the air hole 91 therein can get close to the edge line 22 of the conductive layer, wherein the greatest advantage resulted from the open groove getting close to the edge line 22 of the conductive layer is the reduction of the required sample capacity, which will substantially reduce the requirement on the amount of blood of a person to be tested and reduce the pain of the person to be tested during routine clinical fingertip blood sampling process. That is to say, the solution can ensure the sizes of various electrodes of the sensor in a simpler and more precise way, guarantee the accuracy of the product performance, and achieve further optimization in aspects of the production process and user experiences.

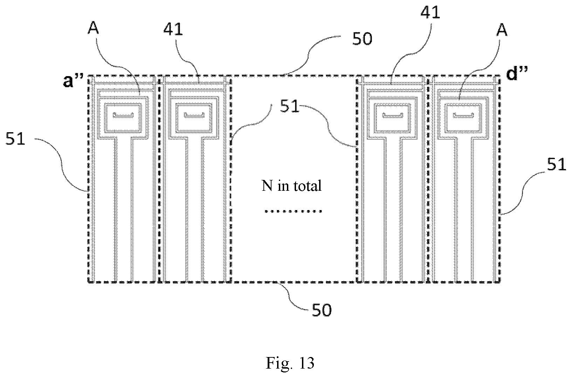

The solution of Fig. 13 is a further improvement of Example 2. Specifically, as shown in Fig. 13, the engraved line 41 for identifying cutting deviation located between the edge line of the conductive layer at the sample contacting end and the electrode and adjacent to the electrode extends to both sides in the transverse direction of the sensor, so that in the process of obtaining the large electrode card by laser etching, the etching path of the engraved line 41 for identifying cutting deviation of a plurality of base units A may travel consecutively. Specifically, as shown in Fig. 13, in the plurality of base units A, the etching path of the engraved line 41 for identifying cutting deviation starts from point a" of the 1st base unit A and finally ends at point d" of the Nth base unit A via the engraved line 41 for identifying cutting deviation of the N base units A. The number of times of jumping of the laser travel path can be reduced in the process of obtaining the large electrode card by laser etching, and more specifically, the optimization of the laser etching production process for the sensor can be achieved, the production efficiency is improved and the time loss is reduced.

### Example 3

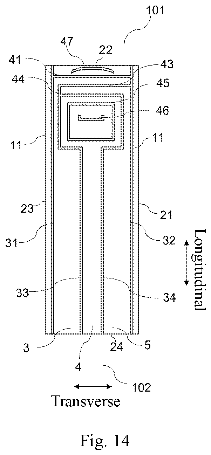

The present invention proposes two technical solutions, in which the electrode system is provided with an engraved line for identifying cutting deviation, to address the risk of fluctuations of the electrode size caused by cutting assembly errors in the production and processing process of the sensor. The quality inspector interprets the engraved line for identifying cutting deviation to determine whether the finished sensor is a qualified product. The interpretability of the engraved line for identifying cutting deviation similarly has an impact on the production efficiency and the rate of finished products. On this basis, this example further optimizes the technical solution of the engraved line for identifying cutting deviation, which will be illustrated below in conjunction with Fig. 14.

As shown in Fig. 14, the engraved line 47 for identifying cutting deviation is disposed between the edge line 22 of the conductive layer at the sample contacting end and the electrode 3, and the engraved line for identifying cutting deviation is a curve. Compared with Example 1, it is easier to interpret the engraved line for identifying cutting deviation in this example. Specifically, the relative physical relationship between the edge line 22 of the conductive layer at the sample contacting end and the engraved line for identifying cutting deviation in Fig. 14 is more diversified compared with that in Fig. 2 of the solution in Example 1, the relative physical relationship between the edge line 22 of the conductive layer and the engraved line for identifying cutting deviation in Fig. 1 is in a parallel state, and the quality inspector may be disturbed when interpreting the level of completeness of the engraved line for identifying cutting deviation, whereas in Fig. 14 of this solution, it is easier and simpler to interpret the completeness of the arc-shaped engraved line for identifying cutting deviation, and less prone to misreading and wrong determination, which is of great help in improving the rate of finished products of the sensor.

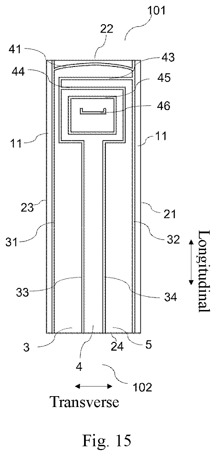

Similarly, compared with the solution of Example 2, in the new technical solution as shown in Fig. 15, it is easier and simpler to interpret the engraved line 41 for identifying cutting deviation, and less prone to misinterpretation and wrong determination, which is of great help in ensuring the quality of finished products of the sensor.

It should be noted that in addition to an arc as shown the figure, the engraved line for identifying cutting deviation in the solution of this example may be other irregular curve such as a wavy line and a meander line, and the embodiment shown here is just an instance and is not necessarily limited to this embodiment.

### Example 4

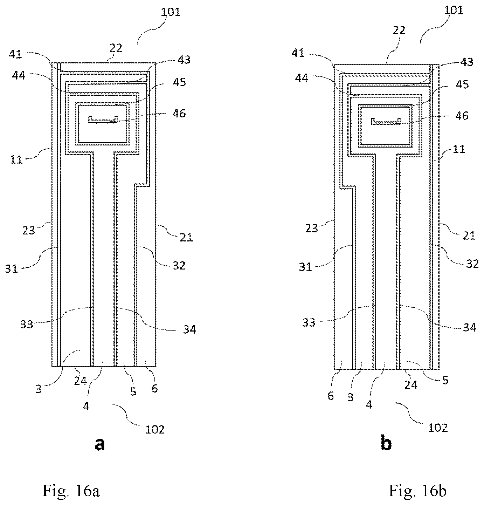

The present invention proposes a technical solution, in which the electrode system is provided with an engraved line for identifying cutting deviation, to address the risk of fluctuations of the electrode size caused by cutting assembly errors in the production and processing process of the sensor. The engraved line for identifying cutting deviation is located between the end edge of the conductive layer at the sample contacting end of the sensor and the electrode, and the electrode refers to an electrode participating in the conduction of an electrical signal. Another special form of this technical solution will be stated below with Fig. 16.

As shown in Fig. 16, the electrode 6 is adjacent to the edge line 22 of the conductive layer at the sample contacting end of the sensor. More specifically, as shown in Fig. 16a, the electrode 6 is enclosed by the engraved lines 31, 41, 32 together with the edge lines 21, 22, 24 of the conductive layer; and as shown in Fig. 16b, the electrode 6 is enclosed by the engraved lines 32, 41, 31 together with the edge lines 23, 22, 24 of the conductive layer. The sensors described in Figs. 16a and 16b represent sales in different market areas through differences in the position where the electrode 6 is disposed, respectively. The electrode 6 in Example 4 does not participate in the conduction of electrical signals, and is only used for differentiation of appearance categories of reagent strips to ensure no cross-boundary selling between different markets. More specifically, as the electrode 6 does not participate in the conduction of electrical signals, fluctuations in the size of the electrode 6 resulted from fluctuations of the edge lines 22 and 24 of the conductive layer caused by the cutting deviation will not produce unqualified products. Only when the size of the electrode 3 is changed under the effect of the cutting deviation, it is considered to be an unqualified product. That is, at this time, the engraved line 41 located between the edge line 22 of the conductive layer at the sample contacting end and the electrode 3 adjacent to the edge line 22 of the conductive layer and participating in the conduction of electrical signals can still be used as the engraved line for identifying cutting deviation. The quality inspector makes determinations on the cut sensors. When a complete engraved line 41 is present, the resultant sensor can be considered as a finished sensor, and when the engraved line 41 is not present or is incomplete, the resultant sensor is considered as an unqualified product.

In other words, when the electrode adjacent to the end edge of the conductive layer at the sample contacting end of the sensor does not participate in the conduction of electrical signals and is used for other purposes, the engraved line located between the end edge of the conductive layer at the sample contacting end of the sensor and the electrode adjacent to the end edge of the conductive layer and participating in the conduction of electrical signals can still be considered as an engraved line for identifying cutting deviation, which is a special form of the solution of the present patent.

### Example 5: glucose test data

The present invention will be further illustrated below in conjunction with the drawings and Fig. 11 of the solution in Example 2.

As shown in Fig. 11, the glucose biosensor in this example includes an insulating substrate 1, a conductive carbon layer 2, a reagent layer 6, an interlayer 8 and an upper cover 9. Specifically, the glucose biosensor of this example includes a conductive carbon coating 2 of uniform thickness, which is prepared by a coating process and located on the insulating substrate 1. Laser engraved lines 31, 32, 33, 34, 41, 42, 43, 44, 45, 46 and the like are etched on the conductive carbon coating using the laser etching technology according to a reasonable programming design, and the conductive layer is removed by ablation of the laser engraved lines to expose the insulating substrate, thus forming electrodes 3, 4, 5 on the conductive layer 2 that are not connected to each other. The reagent layer 6 is located on the electrodes 3, 4, 5 and in a open groove 81 of the interlayer 8. The interlayer 8 is covered with the upper cover 9, and the air hole 91 in the upper cover is located at the bottom of the open groove 81 of the interlayer. The open groove 81 of the interlayer, and the hydrophilic upper cover 9 and the air hole 91 above the open groove form a blood sample injection channel.

After the glucose sensor is inserted into the test instrument, the test instrument is started, the blood sample is sucked into the sample injection channel by siphoning, and the air in the original sample injection channel is discharged through the air hole 91 in the upper cover at the tail end of the sample injection channel, which effectively ensures the smoothness that the blood flows into the channel. When the blood enters to fill in the channel, a DC voltage of 200-500mv is applied to the electrode so that the reagent layer 6 carries out a redox reaction with the glucose to be tested in the blood and generates a test current. The test current is appropriately corrected and compensated for by the test instrument according to the detected current value and the ambient temperature measured by a temperature sensor, and is converted into a glucose value for display to the user.

The materials of the reagent layer 6 mainly include glucose dehydrogenase FAD-GDH, potassium ferricyanide and a second electron enzyme mediator; and the enzyme activity of the glucose dehydrogenase is within the range of 200-600U/mg.

11 blood samples with different glucose concentrations are tested at room temperature using the above glucose sensor. The hematocrits of different concentrations of blood samples are adjusted to 42% ± 2% before the test, and their specific concentrations are obtained through testing with a glucose lactate analyzer (model YSI 2300 STAT) manufactured by YSI Corporation, U.S.A. The test is repeated 10 times for each concentration of blood sample, and the test results are shown in Table 1.

**Table 1 Test results of glucose sensor**

| YSI reading (mg/dl) | 12 | 23 | 52 | 84 | 113 | 166 | 220 | 327 | 446 | 557 | 655 |
|---|---|---|---|---|---|---|---|---|---|---|---|
| Reading 1 | LO | 16 | 44 | 75 | 110 | 169 | 215 | 296 | 417 | 524 | HI |
| Reading 2 | LO | 16 | 45 | 79 | 108 | 170 | 209 | 301 | 405 | 520 | HI |
| Reading 3 | LO | 16 | 45 | 78 | 114 | 160 | 209 | 304 | 408 | 515 | HI |
| Reading 4 | LO | 17 | 44 | 76 | 108 | 162 | 213 | 291 | 422 | 507 | HI |
| Reading 5 | LO | 16 | 47 | 77 | 110 | 163 | 208 | 280 | 414 | 528 | HI |
| Reading 6 | LO | 17 | 46 | 77 | 108 | 166 | 208 | 303 | 425 | 517 | HI |
| Reading 7 | LO | 16 | 46 | 75 | 118 | 157 | 210 | 306 | 432 | 517 | HI |
| Reading 8 | LO | 17 | 46 | 76 | 112 | 169 | 215 | 293 | 413 | 523 | HI |
| Reading 9 | LO | 16 | 46 | 79 | 111 | 163 | 207 | 302 | 413 | 517 | HI |
| Reading 10 | LO | 16 | 46 | 79 | 110 | 161 | 207 | 305 | 416 | 511 | HI |
| Average value | ---- | 16.3 | 45.5 | 77.1 | 110.9 | 164.0 | 210.1 | 298.1 | 416.5 | 517.9 | ---- |
| Standard deviation | ---- | 0.48 | 0.97 | 1.60 | 3.14 | 4.35 | 3.11 | 8.17 | 8.02 | 6.21 | ---- |
| Test precision | ---- | 3.0% | 2.1% | 2.1% | 2.8% | 2.7% | 1.5% | 2.7% | 1.9% | 1.2% | ---- |
| Test deviation | ---- | -6.4 | -6.9 | -6.9 | -1.8% | -1.4% | -4.6% | -8.8% | -6.6% | -7.0% | ---- |

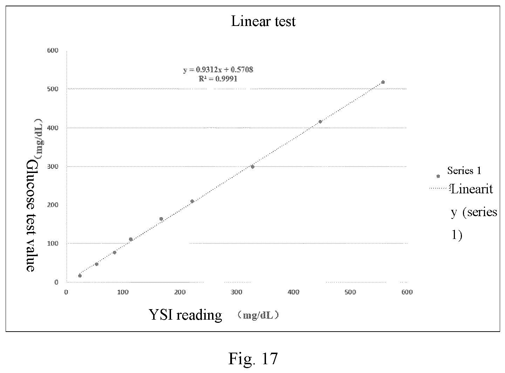

Fig. 17 is a line graph of the glucose sensor test values versus the YSI test values of the example, with a fitting equation y = 0.9312x + 0.5708; and R² = 0.9991. It can be seen that the glucose sensor has a good line shape.

## Claims

1. A biosensor, comprising an insulating substrate and a conductive layer disposed on the insulating substrate, engraved lines and electrodes divided by the engraved lines being distributed on the conductive layer, wherein an engraved line for identifying cutting deviation is disposed at least at a sample contacting end of the biosensor.

2. The biosensor according to claim 1, wherein the engraved line for identifying cutting deviation is located between the end edge of the conductive layer at the sample contacting end and the electrode adjacent to the end edge of the conductive layer at the sample contacting end.

3. The biosensor according to claim 1, wherein the engraved line for identifying cutting deviation is located between the end edge of the conductive layer at the sample contacting end and the electrode closest to the end edge of the conductive layer and participating in the conduction of electrical signals.

4. The biosensor according to one of claims 1 to 3, wherein the engraved line for identifying cutting deviation intersects or does not intersect side edges of the conductive layer.

5. The biosensor according to claim 4, wherein the engraved line for identifying cutting deviation is a straight line parallel to the end edge of the conductive layer or the engraved line for identifying cutting deviation is a curve.

6. The biosensor according to claim 4, wherein the engraved line for identifying cutting deviation does not participate in formation of the electrode.

7. The biosensor according to claim 4, wherein the engraved line for identifying cutting deviation participates in formation of the electrode, and the electrode is adjacent to the end edge of the conductive layer at the sample contacting end.

8. The biosensor according to claim 1, wherein the conductive layer is a gold film, a palladium film, an alloy film or a carbon film.

9. The biosensor according to claim 1, wherein the width of the engraved lines is within the range of 0.020mm to 0.200mm.

10. A preparation method of a biosensor, comprising the following steps:
step 1: according to a pre-designed electrode pattern, performing laser etching on an original material having an insulating substrate and a conductive layer to etch the electrode pattern;
step 2: adding a test reagent to corresponding electrodes;
step 3: affixing an interlayer and an upper cover on the electrodes, and forming a sample injection channel at the sample injection end of the biosensor to obtain a semi-finished large card;
step 4: cutting the semi-finished large card by using a cutter along a preset cutting line to obtain finished biosensors used alone,
wherein the engraved line for identifying cutting deviation according to one of claims 1 to 9 is etched at the sample injection end of the biosensor in step 1
